# EUROPEAN PATENT APPLICATION

(11) **EP 4 613 271 A1**
(43) Date of publication of application: **10.09.2025**
(21) Application number: 23923667.2
(22) Date of filing: 12.09.2023
(51) Int. Cl.: A61K 31/455, A61P 9/00, A61P 9/10, A61P 9/12, A61P 13/12, A61P 3/06, A61P 3/10, A61P 39/06

(54) **USE OF 1-METHYLNICOTINAMIDE IN PREPARATION OF ANTI-VASCULAR CALCIFICATION DRUG**

(30) Priority: 20.02.2023 CN 202310133152
(71) Applicant: The Eight Affiliated Hospital, Sun Yat-Sen University, Shenzhen City, Guangdong Province 518033 (CN)
(72) Inventor: HUANG, Hui, Shenzhen, Guangdong 518033 (CN); ZHU, Huijin, Shenzhen, Guangdong 518033 (CN); CHEN, Yanlian, Shenzhen, Guangdong 518033 (CN); XIE, Chen, Shenzhen, Guangdong 518033 (CN)
(74) Representative: Hafner & Kohl PartmbB
(86) International application number: PCT/CN2023/118284
(87) International publication number: WO 2024/174501

(57) **Abstract**

Use of 1-methylnicotinamide in preparation of an anti-vascular calcification drug. It is found that the small molecule metabolite 1-methylnicotinamide has an effect of inhibiting vascular calcification and its progress, which provides a new idea for treatment of vascular calcification. Moreover, 1-methylnicotinamide has good drug safety and a strong vascular calcification inhibition effect, and can be used as a candidate drug for inhibiting vascular calcification. In addition, 1-methylnicotinamide is a known small molecule metabolite that is soluble in water or DMSO, has good stability, low cytotoxicity and good drug safety, and can be flexibly administrated in various modes such as oral administration and intravenous injection.

## Description

### TECHNICAL FIELD

The present disclosure belongs to the technical field of biomedicine, and in particular, to use of 1-methylnicotinamide in preparation of anti-vascular calcification drugs.

### BACKGROUND

Vascular calcification is commonly found in people with chronic kidney diseases, hypertension, diabetes, hyperlipidemia, and the elderly. Vascular calcification can increase the risk of heart disease, stroke, and atherosclerotic plaque rupture. Therefore, vascular calcification is an important risk factor for increasing the morbidity and mortality of cardiovascular diseases, and seriously affects the quality of life and prognosis of patients. In order to stop or even reverse its progress, vascular calcification has attracted more and more attention from researchers around the world. Depending on the location of occurrence, vascular calcification can be divided into five types: intimal calcification, medial calcification, adventitial calcification, heart valve calcification, and calciphylaxis. The pathogenesis of vascular calcification has not yet been fully elucidated. The current theories to explain the mechanism of vascular calcification mainly include osteogenic differentiation of vascular smooth muscle cells, deficiency of calcification inhibitors, abnormal calcium and phosphate homeostasis, inflammation, oxidative stress, cell apoptosis, autophagy, matrix remodeling, and miRNA regulation. Currently, there is a lack of effective drugs for the prevention and treatment of vascular calcification in clinical practice. Therefore, in-depth research on the pathogenesis of vascular calcification and the development of highly effective drugs that can be used to prevent and treat vascular calcification are of great clinical significance.

1-Methylnicotinamide (MNAM) is a main metabolite of nicotinamide in the body and is biosynthesized in the liver by exogenous niacin and tryptophan via nicotinamide (NA) under the catalysis of nicotinamide N-methyltransferase. According to existing reports, 1-methylnicotinamide plays an important role in anti-thrombosis, anti-inflammation, anti-atherosclerosis, vasodilation, anti-myocardial hypertrophy, and anti-myocardial fibrosis. For example, 1-methylnicotinamide exerts antithrombotic and anti-inflammatory effects by acting directly on vascular endothelial cells, which may partially contribute to the vasoprotective properties of niacin. 1-Methylnicotinamide demonstrates significant anti-atherosclerotic effects in apolipoprotein APOE-/-mice, and this protective effect is associated with improved prostaglandin-2-dependent endothelial function, inhibition of platelet activation, and reduction of local inflammatory burden in plaques and systemic inflammation. However, there are still no reports on whether 1-methylnicotinamide has the effect of anti-vascular calcification. Therefore, it is of great significance to deeply explore the inhibitory effect of 1-methylnicotinamide on vascular calcification and its related mechanisms.

### SUMMARY

An objective of the present disclosure is to provide use of 1-methylnicotinamide in preparation of anti-vascular calcification drugs.

The following technical solution is used in the present disclosure.

In a first aspect of the present disclosure, provided is use of 1-methylnicotinamide or a pharmaceutically acceptable salt thereof in preparation of a drug for preventing and/or treating vascular calcification, where the 1-methylnicotinamide is a compound represented by Formula (I):

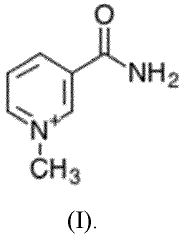

In some embodiments of the present disclosure, the vascular calcification is vascular calcification associated with or caused by chronic kidney diseases, hypertension, diabetes, hyperlipidemia, aging, atherosclerosis, and vascular diseases.

In some embodiments of the present disclosure, the drug further includes a pharmaceutically acceptable excipient.

In some embodiments of the present disclosure, the pharmaceutically acceptable excipient include at least one of a diluent, a binder, a wetting agent, a lubricant, a disintegrant, a solvent, an emulsifier, a cosolvent, a solubilizing agent, a preservative, a pH regulator, an osmotic pressure regulator, a surfactant, a coating material, an antioxidant, an antibacterial agent and a buffer.

In some embodiments of the present disclosure, a dosage form of the drug includes at least one of a suspension, a granule, a capsule, a powder, a tablet, an emulsion, a solution, a pill, an injection, an oral preparation, a suppository, an enema, an aerosol, a patch and a drop.

In some embodiments of the present disclosure, an administration route of the drug includes at least one of intravenous injection, intraperitoneal injection, intramuscular injection, subcutaneous injection, oral administration, sublingual administration, nasal administration, atomization administration and transdermal administration.

In a second aspect of the present disclosure, provided is a drug including 1-methylnicotinamide or a pharmaceutically acceptable salt thereof, where the 1-methylnicotinamide is a compound represented by Formula (I), and the drug is used for preventing and/or treating vascular calcification,

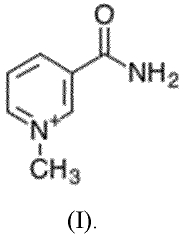

In some embodiments of the present disclosure, the drug further includes an active ingredient for preventing and/or treating vascular calcification.

In some embodiments of the present disclosure, the drug further includes a pharmaceutically acceptable excipient.

In some embodiments of the present disclosure, the pharmaceutically acceptable excipient includes at least one of a diluent, a binder, a wetting agent, a lubricant, a disintegrant, a solvent, an emulsifier, a cosolvent, a solubilizing agent, a preservative, a pH regulator, an osmotic pressure regulator, a surfactant, a coating material, an antioxidant, an antibacterial agent and a buffer.

In some embodiments of the present disclosure, a dosage form of the drug includes at least one of a suspension, a granule, a capsule, a powder, a tablet, an emulsion, a solution, a pill, an injection, an oral preparation, a suppository, an enema, an aerosol, a patch and a drop.

In some embodiments of the present disclosure, an administration route of the drug includes at least one of intravenous injection, intraperitoneal injection, intramuscular injection, subcutaneous injection, oral administration, sublingual administration, nasal administration, atomization administration and transdermal administration.

Beneficial effects of the present disclosure are as follows.

In the present disclosure, it is found that the small molecule metabolite 1-methylnicotinamide has an effect of inhibiting vascular calcification and its progression, which provides a new idea for treatment of vascular calcification. Moreover, 1-methylnicotinamide has good drug safety and a strong vascular calcification inhibitory effect, and can be used as a candidate drug for inhibiting vascular calcification. In addition, 1-methylnicotinamide is a known small molecule metabolite that is soluble in water or dimethyl sulfoxide (DMSO), has good stability, low cytotoxicity, and good drug safety, and can be flexibly administered in various modes such as oral administration and intravenous injection.

### BRIEF DESCRIPTION OF DRAWINGS

Fig. 1 shows that 1-methylnicotinamide inhibits vascular calcification, specifically the results of alizarin red staining in Example 1.
Fig. 2 shows the determination of calcium content in vascular smooth muscle cells in each group in Fig. 1 (**P<0.01).

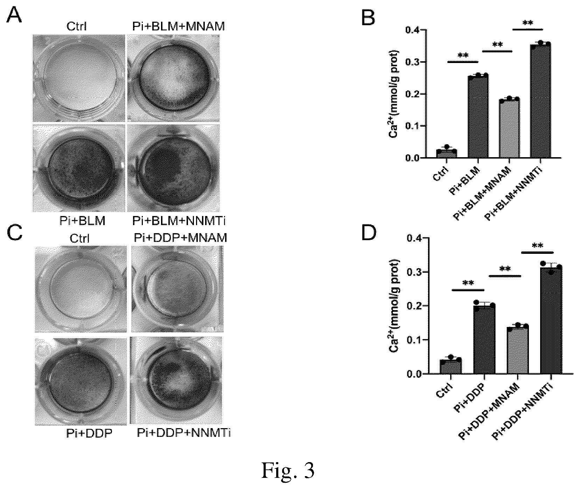

Fig. 3 shows that 1-methylnicotinamide inhibits vascular calcification under aging conditions. Panel A of Fig. 3 shows the results of alizarin red staining, indicating that 1-methylnicotinamide can inhibit the occurrence of calcification of vascular smooth muscle cells induced by bleomycin (BLM) and phosphorus as described in Example 2. Panel B of Fig. 3 shows the determination of calcium content in vascular smooth muscle cells in each group in Panel A of Fig. 3 (**P<0.01). Panel C of Fig. 3 shows the results of alizarin red staining, indicating that 1-methylnicotinamide can inhibit the occurrence of calcification of vascular smooth muscle cells induced by cisplatin (DDP) and phosphorous. Panel D of Fig. 3 shows the determination of calcium content in vascular smooth muscle cells in each group in Panel C of Fig. 3 (**P<0.01).
Fig. 4 shows that 1-methylnicotinamide inhibits vascular calcification in aged mice. Panel A of Fig. 4 shows the results of alizarin red staining in Example 3 (scale: 10 mm). Panel B of Fig. 4 shows the immunohistochemical (IHC) staining results of α-smooth muscle actin (α-SMA) and bone morphogenetic protein 2 (BMP2) in the various mouse arterial rings in Example 3 (scale: 100 µm).

### DETAILED DESCRIPTION

The concepts and technical effects of the present disclosure will be described clearly and completely below in conjunction with embodiments to fully understand the objective, features and effects of the present disclosure. Obviously, embodiments described are only a part of embodiments of the present disclosure, and are not all of embodiments thereof. All other embodiments obtained by those skilled in the art based on the embodiments of the present disclosure without creative efforts shall fall within the scope of protection of the present disclosure.

Human vascular smooth muscle cells (VSMCs) were purchased from iCell Bioscience Inc, Shanghai, China; disodium hydrogen phosphate was purchased from Sigma-Aldrich (Shanghai) Trading Co., Ltd. , China; nicotinamide N-methyltransferase inhibitor (NNMTi) was purchased from Shanghai Topscience Co., Ltd. , China; bleomycin and cisplatin were purchased from MedChemExpress (MCE) , China; the calcium content kit was purchased from Nanjing Jiancheng Bioengineering Institute, China; high-calcium and high-adenine feed was purchased from Guangdong Medical Laboratory Animal Center, China; alizarin red dye was purchased from Beijing Solarbio Science &Technology Co., Ltd. , China; α-SMA primary antibody was purchased from Wuhan Proteintech Group, Inc. , China; BMP2 primary antibody was purchased from Wuhan ABclonal Biotechnology Co., Ltd. , China; and 1-methylnicotinamide (MNAM) was purchased from Sigma-Aldrich (Shanghai) Trading Co., Ltd, China.

### Example 1 1-Methylnicotinamide inhibited vascular calcification

1. In this example, phosphorus was added to induce vascular calcification in vascular smooth muscle cells, and 1-methylnicotinamide or nicotinamide N-methyltransferase inhibitor (NNMTi) was added for intervention for 7 days, and then alizarin red staining was performed to evaluate the inhibitory effect of 1-methylnicotinamide on vascular calcification. The specific procedure was as follows.
   Human vascular smooth muscle cells of passages 8-12 were plated in a 12-well plate at 1×10⁵/well, and 1 mL of DMEM complete medium was added to each well. On the first day after plating, 2.6 mM phosphorus was added to induce calcification, and 100 µM 1-methylnicotinamide or 25 µM nicotinamide N-methyltransferase inhibitor (NNMTi) was added for intervention. On the fourth day, the DMEM complete medium was replaced and 2.6 mM phosphorus was added to induce calcification again, and 100 µM 1-methylnicotinamide or 25 µM nicotinamide N-methyltransferase inhibitor (NNMTi) was added for intervention. On the seventh day, alizarin red staining was performed.
2. To further clarify the inhibitory effect of 1-methylnicotinamide on vascular calcification, phosphorus was added to induce calcification in vascular smooth muscle cells. After 7 days of intervention with 1-methylnicotinamide or nicotinamide N-methyltransferase inhibitor (NNMTi), a calcium content was determined. The specific procedure was as follows.

Human vascular smooth muscle cells of passages 8-12 were plated in a 6-well plate at 2×10⁵/well, and 2 mL of DMEM complete medium was added to each well. On the first day after plating, 2.6 mM phosphorus was added to induce calcification, and 100 µM 1-methylnicotinamide or 25 µM nicotinamide N-methyltransferase inhibitor (NNMTi) was added for intervention. On the fourth day, the DMEM complete medium was replaced and 2.6 mM phosphorus was added to induce calcification again, and 100 µM 1-methylnicotinamide or 25 µM nicotinamide N-methyltransferase inhibitor (NNMTi) was added for intervention. On the seventh day, the calcium content was determined.

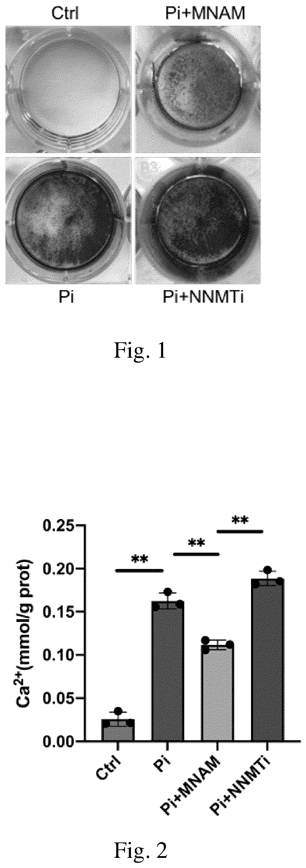

The results of alizarin red staining showed that 1-methylnicotinamide could significantly inhibit the occurrence of vascular calcification, and the inhibitory effect could be reversed by the inhibitor of 1-methylnicotinamide, i.e., N-methyltransferase inhibitor (NNMTi) (Fig. 1). The further calcium content determination results also proved that 1-methylnicotinamide had the effect of inhibiting vascular calcification (Fig. 2).

As could be seen from the above results, the small molecule metabolite 1-methylnicotinamide of Formula (I) could effectively inhibit the occurrence of vascular calcification and could be used as an anti-vascular calcification drug.

### Example 2 1-Methylnicotinamide inhibited vascular calcification under aging conditions

1. In this example, bleomycin (BLM) and phosphorus were added to induce vascular calcification in vascular smooth muscle cells, and 1-methylnicotinamide or nicotinamide N-methyltransferase inhibitor (NNMTi) was added for intervention for 7 days, and then alizarin red staining was performed to evaluate the inhibitory effect of 1-methylnicotinamide on vascular calcification. The specific procedure was as follows.
   Human vascular smooth muscle cells of passages 8-12 were plated in a 12-well plate at 1×10⁵/well, and 1 mL of DMEM complete medium was added to each well. On the first day after plating, 2.6 mM phosphorus was added to induce calcification, and 100 µM 1-methylnicotinamide or 25 µM nicotinamide N-methyltransferase inhibitor (NNMTi) was added for intervention. On the second day, 1 µg/mL bleomycin was added, and on the fourth day, the DMEM complete medium was replaced and 2.6 mM phosphorus was added to induce calcification again, and 100 µM 1-methylnicotinamide or 25 µM nicotinamide N-methyltransferase inhibitor (NNMTi) was added for intervention. On the seventh day, alizarin red staining was performed.
2. To further clarify the inhibitory effect of 1-methylnicotinamide on vascular calcification under the aging conditions, bleomycin and phosphorus were added to induce calcification in vascular smooth muscle cells. After 7 days of intervention with 1-methylnicotinamide or nicotinamide N-methyltransferase inhibitor (NNMTi), a calcium content was determined. The specific procedure was as follows.

Human vascular smooth muscle cells of passages 8-12 were plated in a 6-well plate at 2×10⁵/well, and 2 mL of DMEM complete medium was added to each well. On the first day after plating, 2.6 mM phosphorus was added to induce calcification, and 100 µM 1-methylnicotinamide or 25 µM nicotinamide N-methyltransferase inhibitor (NNMTi) was added for intervention. On the second day, 1 µg/mL bleomycin was added, and on the fourth day, the DMEM complete medium was replaced and 2.6 mM phosphorus was added to induce calcification again, and 100 µM 1-methylnicotinamide or 25 µM nicotinamide N-methyltransferase inhibitor (NNMTi) was added for intervention. On the seventh day, the calcium content was determined.

The results of alizarin red staining showed that 1-methylnicotinamide could significantly inhibit the calcification of bleomycin-induced aged vascular smooth muscle cells, and the inhibitory effect could be reversed by the inhibitor of 1-methylnicotinamide, i.e., N-methyltransferase inhibitor (NNMTi) (Panel A of Fig. 3). The further calcium content determination results also demonstrated that 1-methylnicotinamide had the effect of inhibiting vascular calcification under aging conditions (Panel B of Fig. 3).

3. To further verify the effect of 1-methylnicotinamide in inhibiting vascular calcification under aging conditions, cisplatin (DDP) and phosphorus were added to induce calcification in vascular smooth muscle cells. After 7 days of intervention with 1-methylnicotinamide or nicotinamide N-methyltransferase inhibitor (NNMTi), alizarin red staining was performed to further evaluate the inhibitory effect of 1-methylnicotinamide on vascular calcification under aging conditions. The specific procedure was as follows.

Human vascular smooth muscle cells of passages 8-12 were plated in a 12-well plate at 1×10⁵/well, and 1 mL of DMEM complete medium was added to each well. On the first day after plating, 2.6 mM phosphorus was added to induce calcification, and 100 µM 1-methylnicotinamide or 25 µM nicotinamide N-methyltransferase inhibitor (NNMTi) was added for intervention. On the second day, 1 µg/mL cisplatin was added, and on the fourth day, the DMEM complete medium was replaced and 2.6 mM phosphorus was added to induce calcification again, and 100 µM 1-methylnicotinamide or 25 µM nicotinamide N-methyltransferase inhibitor (NNMTi) was added for intervention. On the seventh day, alizarin red staining was performed.

4. Cisplatin and phosphorus were added to induce calcification in vascular smooth muscle cells, and 1-methylnicotinamide or nicotinamide N-methyltransferase inhibitor (NNMTi) was added for intervention for 7 days, and then the calcium content was determined. The specific procedure was as follows.

Human vascular smooth muscle cells of passages 8-12 were plated in a 6-well plate at 2×10⁵/well, and 2 mL of DMEM complete medium was added to each well. On the first day after plating, 2.6 mM phosphorus was added to induce calcification, and 100 µM 1-methylnicotinamide or 25 µM nicotinamide N-methyltransferase inhibitor (NNMTi) was added for intervention. On the second day, 1 µM cisplatin was added, and on the fourth day, the DMEM complete medium was replaced and 2.6 mM phosphorus was added to induce calcification again, and 100 µM 1-methylnicotinamide or 25 µM nicotinamide N-methyltransferase inhibitor (NNMTi) was added for intervention. On the seventh day, the calcium content was determined.

The results of alizarin red staining showed that 1-methylnicotinamide could significantly inhibit the calcification of cisplatin-induced aged vascular smooth muscle cells, and the inhibitory effect could be reversed by the inhibitor of 1-methylnicotinamide, i.e., N-methyltransferase inhibitor (NNMTi) (Panel C of Fig. 3). The further calcium content determination results also demonstrated that 1-methylnicotinamide had the effect of inhibiting vascular calcification under aging conditions (Panel D of Fig. 3).

As could be seen from the above results, the small molecule metabolite 1-methylnicotinamide of Formula (I) could effectively inhibit the occurrence of vascular calcification under aging conditions and could be used as an anti-vascular calcification drug.

### Example 3 1-Methylnicotinamide inhibited vascular calcification in aged mice

1. In this example, a high-calcium and high-adenine diet was used to induce vascular calcification in aged mice. After intervention by using 1-methylnicotinamide for 2.5 months, alizarin red staining was performed on the aorta of the mice to evaluate the inhibitory effect of 1-methylnicotinamide on vascular calcification in aged mice. The specific procedure was as follows.

Naturally aged C57BL/6 mice (25 months old) were randomly divided into a control group (n=10), a high-calcium and high-adenine group (n=10), and a high-calcium and high-adenine + 1-methylnicotinamide group (100 mg/kg/day) (n=10), and were fed with normal feed, high-calcium and high-adenine feed, and high-calcium and high-adenine feed and 1-methylnicotinamide respectively, and water is normally provided for each group. The mice were killed after 2.5 months, and the aortas of the mice were obtained for alizarin red staining.

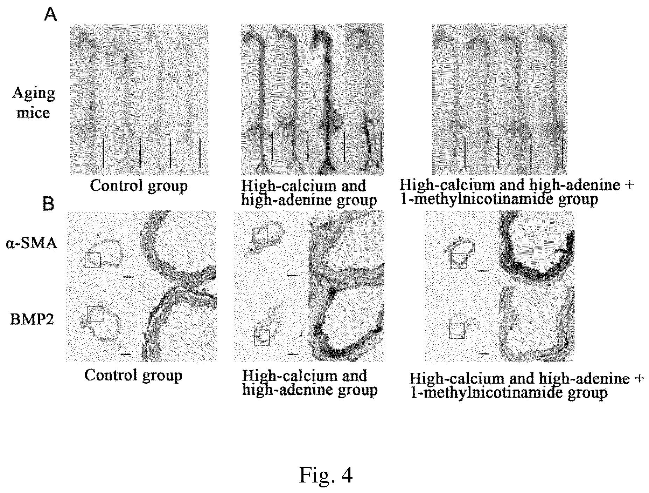

The results of alizarin red staining showed that the high-calcium and high-adenine feed induced vascular calcification successfully in aged mice, while 1-methylnicotinamide could significantly inhibit vascular calcification in aged mice (Panel A of Fig. 4).

2. To further verify the effect of 1-methylnicotinamide in inhibiting vascular calcification in aged mice, a high-calcium and high-adenine diet was used to induce vascular calcification in aged mice. After 2.5 months of intervention with 1-methylnicotinamide, the IHC staining was performed on α-smooth muscle actin (α-SMA) and bone morphogenetic protein 2 (BMP2) in mouse aortic rings to further evalaute the inhibitory effect of 1-methylnicotinamide on vascular calcification in aged mice. The specific procedure was as follows.

Naturally aged C57BL/6 mice (25 months old) were randomly divided into a control group (n=10), a high-calcium and high-adenine group (n=10), and a high-calcium and high-adenine + 1-methylnicotinamide group (n=10), and were fed with normal feed, high-calcium and high-adenine feed, and high-calcium and high-adenine feed and 1-methylnicotinamide respectively, and water is normally provided for each group. The mice were killed after 2.5 months, and the mouse aortic rings were obtained for ICH staining of a-smooth muscle actin (α-SMA) and bone morphogenetic protein 2 (BMP2). The α-SMA was a marker for contractile phenotype vascular smooth muscle cells (VSMCs), while BMP2 was a marker for osteogenic phenotype vascular smooth muscle cells (VSMCs).

The IHC staining results of α-SMA for contractile phenotype VSMCs and BMP2 for osteogenic phenotype VSMCs showed that 1-methylnicotinamide could significantly inhibit vascular calcification in aged mice (Panel B of Fig. 4).

As could be seen from the above results, the small molecule metabolite 1-methylnicotinamide of Formula (I) could effectively inhibit the occurrence of vascular calcification in aged mice and could be used as an anti-vascular calcification drug.

The present disclosure has been described in detail with the foregoing embodiments, but the present disclosure is not limited to these embodiments. Various changes can be made within the knowledge scope of those of ordinary skill in the art without departing from the tenet of the present disclosure. Furthermore, the embodiments of the present disclosure and the features therein may be combined with each other without any conflict.

## Claims

1. Use of 1-methylnicotinamide or a pharmaceutically acceptable salt thereof in preparation of a drug for preventing and/or treating vascular calcification, wherein the 1-methylnicotinamide is a compound represented by Formula (I):

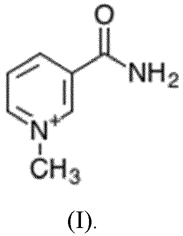

2. The use according to claim 1, wherein the vascular calcification comprises vascular calcification associated with or caused by chronic kidney diseases, hypertension, diabetes, hyperlipidemia, aging, atherosclerosis, and vascular diseases.

3. The use according to any one of claims 1-2, wherein the drug further comprises a pharmaceutically acceptable excipient.

4. The use according to claim 3, wherein the pharmaceutically acceptable excipient comprises at least one of a diluent, a binder, a wetting agent, a lubricant, a disintegrant, a solvent, an emulsifier, a cosolvent, a solubilizing agent, a preservative, a pH regulator, an osmotic pressure regulator, a surfactant, a coating material, an antioxidant, an antibacterial agent and a buffer.

5. The use according to any one of claims 1-2, wherein a dosage form of the drug comprises at least one of a suspension, a granule, a capsule, a powder, a tablet, an emulsion, a solution, a pill, an injection, an oral preparation, a suppository, an enema, an aerosol, a patch and a drop.

6. The use of any one of claims 1-2, wherein an administration route of the drug comprises at least one of intravenous injection, intraperitoneal injection, intramuscular injection, subcutaneous injection, oral administration, sublingual administration, nasal administration, atomization administration and transdermal administration.

7. A drug comprising 1-methylnicotinamide or a pharmaceutically acceptable salt thereof, wherein the 1-methylnicotinamide is a compound represented by Formula (I), and the drug is used for preventing and/or treating vascular calcification;

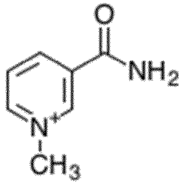

8. The drug according to claim 7, wherein the drug further comprises an active ingredient for preventing and/or treating vascular calcification.
